# EUROPEAN PATENT APPLICATION

(11) **EP 2 286 813 A2**
(43) Date of publication of application: **23.02.2011**
(21) Application number: 10179021.0
(22) Date of filing: 29.01.2007
(51) Int. Cl.: A61K 31/4375, A61P 9/00, A61P 11/00

(54) **Use of naphthyridine derivatives as medicaments**

(30) Priority: 31.01.2006 GB 0601951
(62) Divisional of application: 07703104.5
(71) Applicant: Novartis AG, 4056 Basel (CH)
(72) Inventor: McCarthy, Clive, 4056, Basel (CH); Press, Neil John, Horsham, Sussex RH12 5AB (GB)
(74) Representative: von Sprecher, Georg

(57) **Abstract**

Compounds of formula I 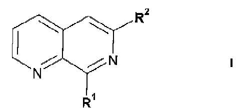 in free or salt form for the preparation of a medicament for the treatment of pulmonary hypertension, where R¹ and R² have the meanings as indicated in the specification. Pharmaceutical compositions that contain the compounds are also described.

## Description

This invention relates to organic compounds and, in particular to their use as pharmaceuticals.

The invention provides, in one aspect, use of a compound of formula I 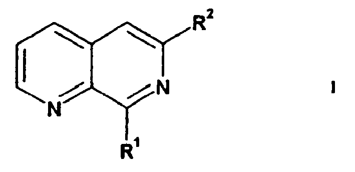 in free or salt form for the preparation of a medicament for the treatment of pulmonary hypertension, where
R¹ is a monovalent aromatic group having up to 10 carbon atoms, and
R² is a cycloaliphatic group having up to 8 ring carbon atoms.

The invention provides, in another aspect, a method of treating pulmonary hypertension, in a subject, particularly a human subject, in need of such treatment, which comprises administering to said subject an effective amount of a compound of formula I as hereinbefore defined.

The invention provides, in further aspect, a method of treating pulmonary hypertension, in a subject, particularly a human subject, in need of such treatment, which comprises administering to said subject an effective amount of a compound of formula I as hereinbefore defined in combination with a second active agent.

Treatment of pulmonary hypertension in accordance with the invention may be symptomatic or prophylactic.

Pulmonary hypertension to be treated in accordance with the invention includes primary pulmonary hypertension (PPH); secondary pulmonary hypertension (SPH); familial PPH; sporadic PPH; precapillary pulmonary hypertension; pulmonary arterial hypertension (PAH); pulmonary artery hypertension; idiopathic pulmonary hypertension; thrombotic pulmonary arteriopathy (TPA); plexogenic pulmonary arteriopathy; functional classes I to IV pulmonary hypertension; and pulmonary hypertension associated with, related to, or secondary to, left ventricular dysfunction, mitral valvular disease, constrictive pericarditis, aortic stenosis, cardiomyopathy, mediastinal fibrosis, anomalous pulmonary venous drainage, pulmonary venoocclusive disease, collagen vasular disease, congenital heart disease, HIV virus infection, drugs and toxins such as fenfluramines, congenital heart disease, pulmonary venous hypertension, chronic obstructive pulmonary disease, interstitial lung disease, sleep-disordered breathing, alveolar hypoventilation disorder, chronic exposure to high altitude, neonatal lung disease, alveolar-capillary dysplasia, sickle cell disease, other coagulation disorder, chronic thromboemboli, connective tissue disease, lupus, schistosomiasis, sarcoidosis or pulmonary capillary hemangiomatosis.

Pulmonary hypertension to be treated in accordance with the invention is most particularly pulmonary hypertension associated with disorders of the respiratory system and/or hypoxemia, including chronic obstructive pulmonary disease, interstitial lung disease, sleep-disordered breathing, alveolar hypoventilation disorders, chronic exposure to high altitude, neonatal lung disease and alveolar-capillary dysplasia, but especially chronic obstructive pulmonary disease.

The terms used in the present specification have the following meanings:
"C₁-C₈-alkyl" as used herein denotes straight chain or branched C₁-C₈-alkyl, which may be, for example, methyl, ethyl, n-propyl, isopropyl, n-butyl, isobutyl, sec-butyl, tert-butyl, straight or branched pentyl, straight or branched hexyl, straight or branched heptyl, or straight or branched octyl. Preferably, C₁-C₈-alkyl is C₁-C₄-alkyl.
"C₁-C₈-alkoxy" as used herein denotes straight chain or branched C₁-C₈-alkoxy which may be, for example, methoxy, ethoxy, n-propoxy, isopropoxy, n-butoxy, isobutoxy, sec-butoxy, tert-butoxy, straight or branched pentoxy, straight or branched hexyloxy, straight or branched heptyloxy, or straight or branched octytoxy. Preferably, C₁-C₈-alkoxy is C₁-C₄-alkoxy.
"C₁-C₈-alkylthio" as used herein denotes straight chain or branched C₁-C₈-alkylthio, which may be, for example, methylthio, ethylthio, n-propylthio, isopropylthio, n-butylthio, isoburylthio, sec-butylthio, tert-burylthio, straight or branched pentylthio, straight or branched hexylthio, straight or branched heptylthio, or straight or branched octylthio. Preferably, C₁-C₈-alkylthio is C₁-C₄-alkylthio.
"C₁-C₈-haloalkyl" as used herein denotes C₁-C₈-alkyl as hereinbefore defined substituted by one or more halogen atoms, preferably one, two or three halogen atoms.
"C₁-C₈-haloalkoxy" as used herein denotes C₁-C₈-alkoxy as hereinbefore defined substituted by one or more halogen atoms, preferably one, two or three halogen atoms.
"C₃-C₈-cycloalkyl" as used herein denotes cycloalkyl having 3 to 8 ring carbon atoms, for example a monocyclic group such as a cyclopropyl, cyclobutyl, cyclopentyl, cyclohexyl, cycloheptyl or cyclooctyl, any of which can be substituted by one or more, usually one or two, C₁-C₄-alkyl groups, or a bicyclic group such as bicycloheptyl or bicyclooctyl. Preferably "C₃-C₈-cycloalkyl" is cyclopropyl, cyclobutyl, cyclopentyl, cyclohexyl, cycloheptyl or cyclooctyl.
"Acyl" as used herein denotes alkylcarbonyl, for example C₁-C₈-alkylcarbonyl where C₁-C₈-alkyl may be one of the C₁-C₈-alkyl groups hereinbefore mentioned, optionally substituted by one or more halogen atoms; cycloalkylcarbonyl, for example C₃-C₈-cycloalkylcarbonyl where C₃-C₈-cycloalkyl may be, for example, cyclopropyl, cyclobutyl, cyclopentyl, cyclohexyl, cycloheptyl or cyclooctyl; 5- or 6- membered heterocyclylcarbonyl having one or two hetero atoms selected from nitrogen, oxygen and sulfur in the ring, such as furylcarbonyl, tetrahydrofurylcarbonyl, pyrrolidinylcarbonyl, piperidinylcarbonyl or pyridylcarbonyl; arylcarbonyl, for example C₆-C₁₀-arylcarbonyl such as benzoyl; or aralkylcarbonyl, for example C₆ to C₁₀-aryl-C₁-C₄-alkylcarbonyl such as benzylcarbonyl or phenylethylcarbonyl.
"C₁-C₈-alkoxycarbonyl" as used herein denotes C₁-C₈-alkoxy as hereinbefore defined linked through an oxygen atom thereof to a carbonyl group.
"C₁-C₈-haloalkoxycarbonyl" as used herein denotes C₁-C₈-haloalkoxy as hereinbefore defined linked through an oxygen atom thereof to a carbonyl group.
"C₁-C₈-hydroxyalkoxycarbonyl" and "C₁-C₈-alkoxy-C₁-C₈alkoxycarbonyl" as used herein denote C₁-C₈-alkoxycarbonyl as hereinbefore defined in which the C₁-C₈-alkoxy group is substituted by hydroxy or a further C₁-C₈-alkoxy group respectively.
"Carboxy-C₁-C₈-alkoxy" as used herein denotes C₁-C₈-alkoxy as hereinbefore defined substituted by carboxy.
"Halogen" or "halo" as used herein may be fluorine, chlorine, bromine or iodine; preferably it is fluorine, chlorine or bromine.

R¹ may be, for example, phenyl optionally substituted by one or more electron-withdrawing substituents, preferably selected from cyano, halogen, carboxy, aminocarbonyl, C₁-C₈-haloalkyl or C₁-C₈-haloalkoxy, preferably one or two such substituents, and/or optionally substituted by C₁-C₈-alkyl, hydroxy, C₁-C₈-alkoxy or C₁-C₈-alkylthio, or R¹ may be a heterocyclic aromatic group having up to 10 ring atoms and 1 to 4 ring hetero atoms, preferably selected from nitrogen, oxygen and sulfur, for example a heterocyclyl group such as furyl, thienyl, pyrrolyl, imidazolyl, pyrazolyl, furazanyl, triazolyl, tetrazolyl, oxazolyl, isoxazolyl, thiazolyl, pyridyl, pyrimidyl, pyridazinyl, triazinyl, indolyl, isoindolyl or benzimidazolyl, which heterocyclyl group may be unsubstituted or substituted e.g. by at least one C₁-C₈-alkyl, halogen or C₁-C₈-alkoxy. Preferred groups R¹ include (a) phenyl substituted by cyano, halogen (particularly fluorine or chlorine), carboxy or C₁-C₄-haloalkoxy, and optionally by C₁-C₄-alkyl or C₁-C₄-alkoxy, (b) phenyl substituted by C₁-C₄-alkoxy and (c) an aromatic heterocyclic group having 5 or 6 ring atoms and one or two ring hetero atoms.

R² may be, for example, a C₃-C₈-cycloalkyl group such as cyclopropyl, methylcyclopropyl, cyclobutyl, cyclopentyl, methytcyclopentyl, cyclohexyl, methylcyclohexyl, dimethylcyclohexyl, cycloheptyl, bicycloheptyl or cyclooctyl, optionally substituted by at least one substituent selected from C₁-C₈-alkyl, C₁-C₈-alkoxy, carboxy, C₁-C₈-alkoxycarbonyl, C₁-C₈-haloalkoxycarbonyl, C₁-C₈-hydroxyalkoxycarbonyl, C₁-C₈-alkoxy-C₁-C₈-alkoxycarbonyl, aminocarbonyl, C₁-C₈-alkylaminocarbonyl, di(C₁-C₈-alkyl)aminocarbonyl, hydroxy, acyl or C₁-C₈-alkyl optionally substituted by hydroxy, cyano, carboxy or C₁-C₈-alkoxycarbonyl. Preferably, R² is C₅-C₇-cycloalkyl substituted by C₁-C₄-alkyl, carboxy, C₁-C₈-alkoxy-carbonyl or aminocarbonyl.

The invention relates to the use of compounds of formula I that preferably include those wherein
R¹ is phenyl substituted by one or two substituents selected from cyano, halogen, carboxy or aminocarbonyl, and optionally by C₁-C₈-alkoxy, or R¹ is phenyl substituted by C₁-C₄-alkoxy, hydroxy or C₁-C₄-alkylthio, and
R² is C₃-C₈-cycloalkyl optionally substituted by at least one substituent selected from C₁-C₄-alkyl, carboxy, C₁-C₈-alkoxycarbonyl or aminocarbonyl.

The invention relates to the use of compounds of formula I that especially include those wherein
R¹ is phenyl substituted by one or two substituents selected from cyano, halogen, carboxy or aminocarbonyl meta to the indicated naphthyridine ring and optionally by C₁-C₄-alkoxy ortho to the indicated naphthyridine ring, or R¹ is phenyl substituted by C₁-C₄-alkoxy meta to the indicated naphthyridine ring, and
R² is C₅-C₇-cycloalkyl optionally substituted by at least one substituent selected from carboxy and C₁-C₄-alkoxycarbonyl.

The compounds represented by formula I are capable of forming acid addition salts, particularly pharmaceutically acceptable acid addition salts. Pharmaceutically acceptable acid addition salts of the compounds of formula I include those of inorganic acids, for example, hydrohalic acids such as hydrofluoric acid, hydrochloric acid, hydrobromic acid or hydroiodic acid, nitric acid, sulfuric acid, phosphoric acid; and organic acids, for example aliphatic monocarboxylic acids such as formic acid, acetic acid, trifluoroacetic acid, propionic acid and butyric acid, aliphatic hydroxy acids such as lactic acid, citric acid, tartaric acid or malic acid, dicarboxylic acids such as maleic acid or succinic acid, aromatic carboxylic acids such as benzoic acid, p-chlorobenzoic acid, diphenylacetic acid or triphenylacetic acid, aromatic hydroxy acids such as o-hydroxybenzoic acid, p-hydroxybenzoic acid, 1-hydroxynaphthalene-2-carboxylic acid or 3-hydroxynaphthalene-2-carboxylic acid, and sulfonic acids such as methanesulfonic acid or benzenesulfonic acid. These salts may be prepared from compounds of formula I by known salt-forming procedures.

Compounds of formula I which contain acidic, e.g. carboxyl, groups, are also capable of forming salts with bases, in particular pharmaceutically acceptable bases such as those well known in the art; suitable such salts include metal salts, particularly alkali metal or alkaline earth metal salts such as sodium, potassium, magnesium or calcium salts, or salts with ammonia or pharmaceutically acceptable organic amines or heterocyclic bases such as ethanolamines, benzylamines or pyridine. These salts may be prepared from compounds of formula I by known salt-forming procedures.

Specific especially preferred compounds of formula I include
4-[8-(3-Cyano-phenyl)-[1,7]naphthyridin-6-yl]-cyclohexanecarboxylic acid ethyl ester;
4-[8-(3-Cyano-phenyl)-[1,7]naphthyridin-6-yl]-cyclohexanecarboxylic acid;
4-[8-(3-Cyano-phenyl)-[1,7]naphthyridin-6-yl]-cyclohexanecarboxylic acid potassium salt;
4-(8-(3-Carbamoyl-phenyl)-[1,7]naphthyridin-6-yl]-cyclohexanecarboxylic acid;
3-[6-(4-Carboxy-cyclohexyl)-[1,7]naphthyridin-8-yl)-benzoic acid;
4-[8-(5-Fluoro-2-methoxy-phenyl)-[1,7]naphthyridin-6-yl]-cyclohexane-carboxylic acid ethyl ester;
4-[8-(5-Fluoro-2-methoxy-phenyl)-[1,7]naphthyridin-6-yl]-cyclohexane-carboxylic acid;
4-[8-(5-Fluoro-2-methoxy-phenyl)-[1,7]naphthyridin-6-yl)-cyclohexanecarbox-ylic acid sodium salt;
4-[8-(3-Methoxy-phenyl)-[1,7]naphthyridin-6-yl]-cyclohexanecarboxylic acid;
Potassium [4-[8-(3-cyano-phenyl)-[1,7] naphthyridin-6-yl]-cyclohexyloxy]-acetate;
4-[8-(3-Fluorophenyl)-[1,7]naphthyridin-6-yl]-cyclohexanecarboxylic acid;
4-[8-(3-Trifluoromethoxyphenyl)-[1,7]naphthyridin-6-yl]-cyclohexane-carboxylic acid;
4-[8-(3-Methylsulfanyl-phenyl)-[1,7]naphthyridin-6-yl]-cyclohexanecarboxylic acid; and
4-[8-(3-Methanesulfinyl-phenyl)-[1,7]naphthyridin-6-yl]-cyclohexanecarboxylic acid.

The invention relates most importantly to the use of 4-[8-(3-Fluorophenyl)-[1,7]naphthyridin-6-yl]-cyclohexanecarboxylic acid, i.e. a compound of formula II

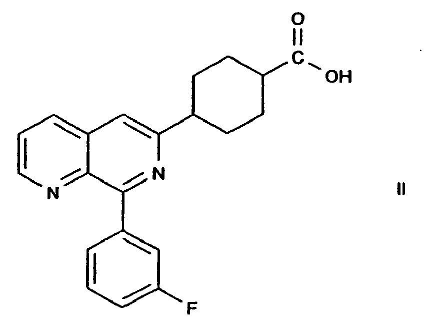

Compounds of formula I, in free or salt form, exhibit cyclic nucleotide phosphodiesterase (PDE) isoenzyme inhibiting activity, selective for type 4 isoenzyme. They possess anti-inflammatory, anti-airways hyperreactivity and bronchodilator properties. They further possess immunosuppressive and TNFα secretion inhibitory activities.

Compounds of formula I, in free or salt form, may be prepared as described in WO 03/039544, the contents of which is incorporated herein by reference.

The compounds of formula I in free or salt form may exist in stereoisomeric forms according to the orientation of moieties attached to the cycloaliphatic ring. The invention embraces both individual such stereoisomers, i.e. cis and trans isomers, as well as mixtures thereof. Where R¹ or R² contain an asymmetric carbon atom, the compounds of formula I in free or salt form exist in individual optically active isomeric forms or as mixtures thereof, e.g. as racemic or diastereomeric mixtures. The invention embraces the use of both individual optically active R and S isomers as well as mixtures, e.g. racemic or diastereomeric mixtures, thereof.

Mixtures of diastereoisomers obtainable in accordance with the process disclosed in WO 03/039544 or by some other method can be separated in customary manner into mixtures of enantiomers, for example racemates, or into individual diastereoisomers, for example on the basis of the physico-chemical differences between the constituents in known manner by fractional crystallisation, distillation and/or chromatography. Advantageously the more active isomer is isolated.

The invention includes all pharmaceutically acceptable isotopically-labelled compounds of formula I wherein one or more atoms are replaced by atoms having the same atomic number, but an atomic mass or mass number different from the atomic mass or mass number usually found in nature. Examples of isotopes suitable for inclusion in the compounds of the invention include isotopes of hydrogen e.g. ²H and ³H, carbon e.g. ¹¹C, ¹³C and ¹⁴C, chlorine e.g. ³⁶Cl, fluorine e.g. ¹⁸F, iodine e.g. ¹²³I and ¹²⁵I, nitrogen e.g. ¹³N and ¹⁵N, oxygen e.g. ¹⁵O, ¹⁷O and ¹⁸O, and sulfur e.g. ³⁵S.

Certain isotopically-labelled compounds of formula I, for example those incorporating a radioactive isotope, are useful in drug and/or substrate tissue distribution studies. The radioactive isotopes tritium (³H) and carbon-14 (¹⁴C) are particularly useful for this purpose in view of their ease of incorporation and ready means of detection. Substitution with heavier isotopes such as deuterium (²H) may afford certain therapeutic advantages that result from greater metabolic stability, for example increased *in vivo* half-life or reduced dosage requirements, and hence may be preferred in some circumstances. Substitution with positron emitting isotopes, such as ¹¹C, ¹⁸F, ¹⁵O, and ¹³N can be useful in Positron Emission Topography (PET) studies for examining substrate receptor occupancy.

Isotopically-labelled compounds of formula I can generally be prepared by conventional techniques known to those skilled in the art or by processes analogous to those described in the accompanying examples using an appropriate isotopically-labelled reagent in place of the non-labelled reagent previously used.

Pharmaceutically acceptable solvates in accordance with the invention include those wherein the solvent of crystallisation may be isotopically substituted e.g. D₂O, d₆-acetone or d₆-DMSO.

The invention provides a method of treating pulmonary hypertension, in a subject, particularly a human subject, in need of such treatment, which comprises administering to said subject an effective amount of a compound of formula I as hereinbefore defined.

The invention also provides a method of treating pulmonary hypertension, in a subject, particularly a human subject, in need of such treatment, which comprises administering to said subject an effective amount of a compound of formula I as hereinbefore defined in combination with a second or additional active agent. Examples of second or additional active agents include, but are not limited to, antiinflammatories, bronchodilators, antihistamines, decongestants, anti-tussives, anticoagulants, diuretics, cardiac glycosides, calcium channel blockers, vasodilators, prostacyclin analogues, endothelin antagonists, phosphodiesterase inhibitors, endopeptidase inhibitors, lipid lowering agents, thromboxane inhibitors, or other agents found, for example, in the *Physician's Desk Reference* 2003. Second active agents can be large molecules (e.g., proteins) or small molecules (e.g., synthetic inorganic, organometallic, or organic molecules). Compounds of formula I may be mixed with second or additional active agents in a fixed pharmaceutical composition or they may be administered separately, before, simultaneously with or after the second or additional active agents. Examples of specific second active agents include, but are not limited to, amlodipine, diltiazem, nifedipine, adenosine, epoprostenol (Floran™), treprostinil (Remodulin™), bosentan (Tracleer™), warfarin, digoxin, nitric oxide, L- arginine, iloprost, betaprost, and sildenafil (Viagra™).

Such anti-inflammatory drugs include steroids, in particular glucocorticosteroids such as budesonide, beclamethasone dipropionate, fluticasone propionate, ciclesonide or mometasone furoate, or steroids described in WO 02/88167, WO 02/12266, WO 02/100879, WO 02/00679 (especially those of Examples 3,11, 14, 17, 19, 26, 34, 37, 39, 51, 60, 67, 72, 73, 90, 99 and 101), WO 03/35668, WO 03/48181, WO 03/62259, WO 03/64445, WO 03/72592, WO 04/39827 and WO 04/66920; non-steroidal glucocorticoid receptor agonists, such as those described in DE 10261874, WO 00/00531, WO 02/10143, WO 03/82280, WO 03/82787, WO 03/86294, WO 03/104195, WO 03/101932, WO 04/05229, WO 04/18429, WO 04/19935, WO 04/26248 and WO 05/05452; LTB4 antagonists such as BIIL 284, CP-195543, DPC11870, LTB4 ethanolamide, LY 293111, LY 255283, CGS025019C, CP-195543, ONO-4057, SB 209247, SC-53228 and those described in US 5451700 and WO 04/108720; LTD4 antagonists such as montelukast, pranlukast, zafirlukast, accolate, SR2640, Wy-48,252, ICI 198615, MK-571, LY-171883, Ro 24-5913 and L-648051; Dopamine receptor agonists such as cabergoline, bromocriptine, ropinirole and 4-hydroxy-7-[2-[[2-[[3-(2-phenylethoxy)-propyl]sulfonyl]ethyl]amino]ethyl]-2(3H)-benzothiazolone and pharmaceutically acceptable salts thereof (the hydrochloride being Viozan^{®}- AstraZeneca); A2a agonists such as those described in EP 409595A2, EP 1052264, EP 1241176, WO 94/17090, WO 96/02543, WO 96/02553, WO 98/28319, WO 99/24449, WO 99/24450, WO 99/24451, WO 99/38877, WO 99/41267, WO 99/67263, WO 99/67264, WO 99/67265, WO 99/67266, WO 00/23457, WO 00/77018, WO 00/78774, WO 01/23399, WO 01/27130, WO 01/27131, WO 01/60835, WO 01/94368, WO 02/00676, WO 02/22630, WO 02/96462, WO 03/086408, WO 04/039762, WO 04/039766, WO 04/045618 and WO 04/046083; and A2b antagonists such as those described in WO 02/42298 and WO 03/042214.

Suitable PDE5 inhibitors include pyrazolopyrimidinones disclosed in EP 463756, EP 526004, WO 94/28902, WO 96/16657 or WO 98/49166; 5-substituted pyrazole [4,3-d]pyrimidin-7-ones disclosed in EP 201188; griseolic acid derivatives disclosed in EP 214708 and EP 319050; 2-phenylpurinone derivatives disclosed in EP 293063; phenylpyridone derivatives disclosed in EP 347027; fused pyrimidine derivatives disclosed in EP 347146; condensed pyrimidine derivatives disclosed in EP 349239; pyrimidopyrimidine derivatives disclosed in EP 351058; purine compounds disclosed in EP 352960; quinazolinone derivatives disclosed in EP 371731; phenylpyrimidone derivatives disclosed in EP 395328; imidazoquin-oxalinone derivatives or their aza analogues disclosed in EP 400583; phenylpyrimidone derivatives disclosed in EP 400799; phenylpyridone derivatives disclosed in EP 428268; pyrimidopyrimidine derivatives disclosed in EP 442204; 4-aminoquinazoline derivatives disclosed in EP 579496; 4,5-dihydro-4-oxo-pyrrolo[1,2-a]quinoxaline derivatives or their aza analogues disclosed in EP 584487; polycyclic guanine derivatives disclosed in WO91/1971; nitrogenous heterocyclic compounds disclosed in WO93/07124; 2-benzyl-polycyclic guanine derivatives disclosed in WO94/19351; quinazoline derivatives disclosed in US 4060615; 6-heterocyclyl pyrazolo [3,4-d]pyrimidin-4-ones disclosed in US 5294612; benzimidazoles disclosed in Japanese Kokai 5-222000; cycloheptimidazoles disclosed in European Journal of Pharmacology, vol. 251, (1994), 1; N-containing heterocycles disclosed in WO 94/22855; pyrazolopyrimidine derivatives disclosed in EP 636626; 4-aminopyrimidine derivatives disclosed in EP 640599; imidazoquinazoline derivatives disclosed in EP 668280; anthranilic acid derivatives disclosed in EP 0686625; 4-aminoquinazoline derivatives disclosed in US 5436233; tetracyclic derivatives disclosed in WO 95/19978 (including tadafil); quinazoline compounds disclosed in EP 0669324; fused pyridazine compounds disclosed in EP 722936; imidazoquinoline compounds disclosed in EP 0758653; substituted pyrazoloquinolinamines disclosed in WO 96/28159; substituted pyrazolopyrimidinones disclosed in WO 96/28429; indole derivatives disclosed in WO 96/32379; benzimidazole derivatives disclosed in WO 97/03070; 2-phenyl substituted imidazotriazinone derivatives disclosed in WO 99/24433 (including vardenafil); as well as those described in WO 2001/77110.

Although compounds of formula I are PDE4 inhibitors the second or additional agent could also be a PDE4 inhibitor such as cilomilast (Ariflo® GlaxoSmithKline), Roflumilast (Byk Gulden),V-11294A (Napp), BAY19-8004 (Bayer), SCH-351591 (Schering-Plough), Arofylline (Almirall Prodesfarma), PD189659 / PD168787 (Parke-Davis), AWD-12-281 (Asta Medica), CDC-801 (Celgene), SelCID(TM) CC-10004 (Celgene), VM554/UM565 (Vernalis), T-440 (Tanabe), KW-4490 (Kyowa Hakko Kogyo), GRC 3886 (Oglemilast, Glenmark), WO 92/19594, WO 93/19749, WO 93/19750, WO 93/19751, WO 99/16766, WO 01/13953, WO 03/104204, WO 03/104205, WO 04/000814, WO 04/000839 and WO 04/005258 (Merck), WO 04018450, WO 04/018451, WO 04/018457, WO 04/018465, WO 04/018431, WO 04/018449, WO 04/018450, WO 04/018451, WO 04/018457, WO 04/018465, WO 04/019944, WO 04/019945, WO 04/045607, WO 04/037805, WO 04/063197, WO 04/103998, WO 04/111044, WO 05012252, WO 05012253, WO 05/013995, WO 05/030212, WO 05/030725, WO 05/087744, WO 05/087745, WO 05/087749 and WO 05/090345 as well as those described in WO 98/18796 and WO 03/39544.

Such bronchodilatory drugs include beta-2 adrenoceptor agonists. Suitable beta-2 adrenoceptor agonists include albuterol (salbutamol), metaproterenol, terbutaline, salmeterol, fenoterol, procaterol, and especially, formoterol, carmoterol, GSK159797 and pharmaceutically acceptable salts thereof, and compounds (in free or salt or solvate form) of formula I of WO 0075114, which document is incorporated herein by reference, preferably compounds of the Examples thereof, especially a compound of formula 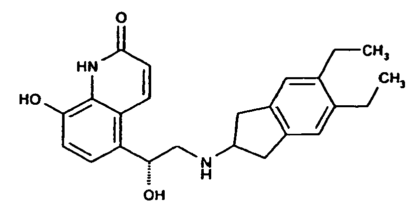 and pharmaceutically acceptable salts thereof, as well as compounds (in free or salt or solvate form) of formula I of WO 04/16601 or of formula I of WO 04/087142. Further suitable β -2-adrenoreceptor agonists include compounds, such as those described in and also compounds of EP 147719, EP 1440966, EP 1460064, EP 1477167, EP 1574501, JP 05025045, JP 2005187357, US 2002/0055651, US 2004/0242622, US 2004/0229904, US 2005/0133417, US 2005/5159448, US 2005/5159448, US 2005/171147, US 2005/182091, US 2005/182092, US 2005/209227, US 2005/256115, US 2005/277632, US 2005/272769, US 2005/239778, US 2005/215542, US 2005/215590, US 2006/19991, US 2006/58530, WO 93/18007, WO 99/64035, WO 01/42193, WO 01/83462, WO 02/66422, WO 02/ 70490, WO 02/76933, WO 03/24439, WO 03/42160, WO 03/42164, WO 03/72539, WO 03/91204, WO 03/99764, WO 04/16578, WO 04/22547, WO 04/32921, WO 04/33412, WO 04/37768, WO 04/37773, WO 04/37807, WO 04/39762, WO 04/39766, WO 04/45618 WO 04/46083 , WO 04/80964, WO 04/087142, WO 04/89892, WO 04/108675, WO 04/108676, WO 05/33121, WO 05/40103, WO 05/44787, WO 05/58867, WO 05/65650, WO 05/66140, WO 05/70908, WO 05/74924, WO 05/77361, WO 05/90288, WO 05/92860, WO 05/92887, WO 05/90287, WO 05/95328, WO 05/102350, WO 06/56471, WO 06/74897 or WO 06/8173.

Such bronchodilatory drugs also include other anticholinergic or antimuscarinic agents, in particular ipratropium bromide, oxitropium bromide, tiotropium salts, glycopyrrolate, CHF 4226 (Chiesi) and SVT-40776, but also those described in EP 424021, US 3714357, US 5171744, US 2005/171147, US 2005/182091, WO 01/04118, WO 02/00652, WO 02/51841, WO 02/53564, WO 03/00840, WO 03/33495, WO 03/53966, WO 03/87094, WO 04/18422, WO 04/05285, WO 04/96800, WO 05/77361 and WO 06/48225. Suitable dual anti-inflammatory and bronchodilatory drugs include dual beta-2 adrenoceptor agonist / muscarinic antagonists such as those disclosed in US 2004/0167167, US 2004/0242622, US 2005/182092, US 2005/256114, US 2006/35933, WO 04/74246, WO 04/74812, WO 04/89892 and WO 06/23475.

Suitable antihistamine drug substances include cetirizine hydrochloride, levocetirizine, acetaminophen, clemastine fumarate, promethazine, loratidine, desloratidine, diphenhydramine and fexofenadine hydrochloride, activastine, astemizole, azelastine, dimetinden, ebastine, epinastine, levocabastine, mizolastine and tefenadine as well as those disclosed in WO 03/099807, WO 04/026841 and JP 2004107299.

In the treatment of disorders in accordance with the invention, compounds of formula I, in free form or in pharmaceutically acceptable salt form, may be administered by any appropriate route, for example orally, e.g. in tablet, capsule or liquid form, parenterally, for example in the form of an injectable solution or suspension, or intranasally, for example in the form of an aerosol or other atomisable formulation using an appropriate intranasal delivery device, e.g. a nasal spray such as those known in the art, or by inhalation.

A compound of formula I or II in free base or acid addition salt form may be administered in a pharmaceutical composition together with a pharmaceutically acceptable diluent or carrier. Such compositions may be as described in WO 03/039544, for example dry powders, tablets, capsules and liquids, but also injection solutions, infusion solutions or inhalation suspensions, which may be prepared using other formulating ingredients and techniques known in the art.

The dosage of the compound of formula I or II in free base or acid addition salt form can depend on various factors, such as the activity and duration of action of the active ingredient, the severity of the condition to be treated, the mode of administration, the species, sex, ethnic origin, age and weight of the subject and/or its individual condition.

In one embodiment of the invention, the recommended daily dose range of the compound of formula for the conditions described herein lie within the range of from about I mg to about 10,000 mg per day, given as a single once-a-day dose, or in divided doses throughout a day. Specifically, a daily dose range should be from about 1 mg to about 5,000 mg per day, more specifically, between about 10 mg and about 2,500 mg per day, between about 100 mg and about 800 mg per day, between about 100 mg and about 1,200 mg per day, or between about 25 mg and about 2,500 mg per day. In managing the patient, the therapy should be initiated at a lower dose, perhaps about 1 mg to about 2,500 mg, and increased if necessary up to about 200 mg to about 5,000 mg per day as either a single dose or divided doses, depending on the patient's global response. In another embodiment of the invention, a compound of formula I is administered from about 1 to about 20 mg/day individually, for example, about 1 mg/day, about 2 mg/day, about 3 mg/day, about 4 mg/day, about 5 mg/day, about 6 mg/day, about 7 mg/day, about 8 mg/day, about 9 mg/day, about 10 mg/day, about 11 mg/day, about 12 mg/day, about 13 mg/day, about 14 mg/day, about 15 mg/day, about 16 mg/day, about 17 mg/day, about 18 mg/day, about 19 mg/day, or about 20 mg/day. In a particular embodiment, 4-[8-(3-Fluorophenyl)-[1,7]naphthyridin-6-yl]-cyclohexanecarboxylic acid is administered in an amount of about 400, 800, 1,200, 2, 500, 5,000 or 10,000 mg a day in a single dose or as two divided doses.

Administration of a compound of formula I and a second active agent to a patient can occur simultaneously or sequentially by the same or different routes of administration. The suitability of a particular route of administration employed for a particular active agent will depend on the active agent itself (e.g., whether it can be administered orally without decomposing prior to entering the blood stream) and the disease being treated. A preferred route of administration for a compound of formula I is oral. Another preferred route of administration for such a compound is parenteral, particularly for patients who are in a peritransplant period or in an end stage of pulmonary hypertension. Preferred routes of administration for the second active agent of the invention are known to those of ordinary skill in the art such as in Physicians' Desk Reference (57th ed., 2003).

The specific amount of the second active agent will depend on the specific agent used, the type of pulmonary hypertension being treated or managed, the severity and stage of pulmonary hypertension, and the amount(s) of any optional additional active agents concurrently administered to the patient.

This present invention encompasses kits which, when used by the medical practitioner, can simplify the administration of appropriate amounts of active agents to a patient. A typical kit of the invention comprises a dosage form of a compound of formula I, in free or salt form, optionally further comprising a second active agent and/or a device or devices for administering the active agents e.g. syringes, drip bags, patches or inhalers.

The invention is illustrated by the following Example.

### EXAMPLE

The compound of formula II, namely 4-[8-(3-Fluorophenyl)-[1,7]naphthyridin-6-yl]-cyclohexanecarboxylic acid and being a representative example of compounds of formula I, is tested in the monocrotaline reversal model of pulmonary hypertension that is described by Schermuly et al in The Journal of Clinical Investigation 2005 Oct; 115(10):2811-21. The disclosure of that article is incorporated herein by way of reference.

4-[8-(3-Fluorophenyl)-[1,7]naphthyridin-6-yl]-cyclohexanecarboxylic acid is dosed orally to the animals once daily from days 21-42 of the experiment at 0.3, 1 and 3 mg/kg. The compound shows efficacy at 3 mg/kg in all parameters tested, including: (i) reversal of RV (right ventricular) pressure; (ii) reversal of RV hypertrophy (iii) medial wall thickness; and (iv) mortality.

## Claims

1. The use of a compound of formula I 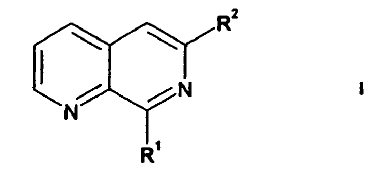 in free or salt form for the preparation of a medicament for the treatment of pulmonary hypertension, where
R¹ is a monovalent aromatic group having up to 10 carbon atoms, and
R² is a cycloaliphatic group having up to 8 ring carbon atoms.

2. Use according to claim 1, wherein
R¹ is phenyl substituted by one or two substituents selected from cyano, halogen, carboxy or aminocarbonyl, and optionally by C₁-C₈-alkoxy, or R¹ is phenyl substituted by C₁-C₄-alkoxy, hydroxy or C₁-C₄-alkylthio, and
R² is C₃-C₈-cycloalkyl optionally substituted by at least one substituent selected from C₁-C₄-alkyl, carboxy, C₁-C₈-alkoxycarbonyl or aminocarbonyl.

3. Use according to claim 1 or 2, wherein
R¹ is phenyl substituted by one or two substituents selected from cyano, halogen, carboxy or aminocarbonyl meta to the indicated naphthyridine ring and optionally by C₁-C₄-alkoxy ortho to the indicated naphthyridine ring, or R¹ is phenyl substituted by C₁-C₄-alkoxy meta to the indicated naphthyridine ring, and
R² is C₅-C₇-cycloalkyl optionally substituted by at least one substituent selected from carboxy and C₁-C₄-alkoxycarbonyl.

4. Use according to any preceding claim, wherein the compound of formula I is selected from the group consisting of:
4-[8-(3-Cyano-phenyl)-[1,7]naphthyridin-6-yl]-cyclohexanecarboxylic acid ethyl ester;
4-[8-(3-Cyano-phenyl)-[1,7]naphthyridin-6-yl]-cyclohexanecarboxylic acid;
4-[8-(3-Cyano-phenyl)-[1,7]naphthyridin-6-yl]-cyclohexanecarboxylic acid potassium salt;
4-[8-(3-Carbamoyl-phenyl)-[1,7]naphthyridin-6-yl]-cyclohexanecarboxylic acid;
3-[6-(4-Carboxy-cyclohexyl)-[1,7]naphthyridin-8-yl]-benzoic acid;
4-[8-(5-Fluoro-2-methoxy-phenyl)-[1,7]naphthyridin-6-yl]-cyclohexane-carboxylic acid ethyl ester;
4-[8-(5-Fluoro-2-methoxy-phenyl)-[1,7]naphthyridin-6-yl]-cyclohexane-carboxylic acid;
4-[8-(5-Fluoro-2-methoxy-phenyl)-[1,7]naphthyridin-6-yl]-cyclohexanecarbox-ylic acid sodium salt;
4-[8-(3-Methoxy-phenyl)-[1,7]naphthyridin-6-yl]-cyclohexanecarboxylic acid;
Potassium {4-[8-(3-cyano-phenyl)-[1,7] naphthyridin-6-yl]-cyclohexyloxy}-acetate;
4-[8-(3-Fluorophenyl)-[1,7]naphthyridin-6-yl]-cyclohexanecarboxylic acid;
4-[8-(3-Trifluoromethoxyphenyl)-[1,7]naphthyridin-6-yl]-cyclohexane-carboxylic acid;
4-[8-(3-Methylsulfanyl-phenyl)-[1,7]naphthyridin-6-yl]-cyclohexanecarboxylic acid; and
4-[8-(3-Methynesulfinyl-phenyl)-[1,7]naphthyridin-6-yl]-cyclohexanecarboxylic acid.

5. Use according to any preceding claim, wherein the compound of formula I is 4-[8-(3-Fluorophenyl)-[1,7]naphthyridin-6-yl]-cyclohexanecarboxylic acid in free or salt form.

6. Use according to any one of claims 1 to 5, in combination with a second active agent selected from the group consisting of antiinflammatories, bronchodilators, antihistamines, decongestants, anti-tussives, anticoagulants, diuretics, cardiac glycosides, calcium channel blockers, vasodilators, prostacyclin analogues, endothelin antagonists, phosphodiesterase inhibitors, endopeptidase inhibitors, lipid lowering agents and thromboxane inhibitors.

7. Use according to any one of claims 1 to 6, in which the pulmonary hypertension is primary pulmonary hypertension.

8. Use according to any one of claim 1 to 6, in which the pulmonary hypertension is associated with chronic obstructive pulmonary disease.

9. The use of 4-[8-(3-Fluorophenyl)-[1,7]naphthyridin-6-yl]-cyclohexanecarboxylic acid in free or salt form for the preparation of a medicament for the treatment of primary pulmonary hypertension.

10. The use of 4-[8-(3-Fluorophenyl)-[1,7]naphthyridin-6-yl]-cyclohexanecarboxylic acid in free or salt form for the preparation of a medicament for the treatment of primary pulmonary hypertension associated with chronic obstructive pulmonary disease.
